# EUROPÄISCHE PATENTSCHRIFT

(11) **EP 4 272 777 B1**
(45) Veröffentlichungstag und Bekanntmachung des Hinweises auf die Patenterteilung: **24.06.2026**
(21) Anmeldenummer: 23170331.5
(22) Anmeldetag: 27.04.2023
(51) Int. Cl.: A61M 5/14, A61M 5/142, A61M 5/168, A61M 39/08

(54) **INFUSIONSSYSTEM UND KATHETER FÜR EIN SOLCHES INFUSIONSSYSTEM**
INFUSION SYSTEM AND CATHETER FOR SUCH AN INFUSION SYSTEM
SYSTÈME DE PERFUSION ET CATHÉTER POUR UN TEL SYSTÈME DE PERFUSION

(30) Priorität: 05.05.2022 DE 102022204439
(43) Veröffentlichungstag der Anmeldung: 08.11.2023
(73) Patentinhaber: B. Braun Melsungen AG, 34212 Melsungen (DE)
(72) Erfinder: Schröder, Tobias, 34212 Melsungen (DE); Weiß, André, 34302 Guxhagen (DE); Wildhagen, Jens, 30659 Hannover (DE)
(74) Vertreter: Patentanwälte Ruff, Wilhelm, Beier, Dauster & Partner mbB

(56) Entgegenhaltungen:
- DE-B4- 102004 047 195
- US-A1- 2004 171 985
- US-A1- 2009 318 902

## Beschreibung

Die Erfindung betrifft ein Infusionssystem zum Abgeben unterschiedlicher medizinischen Flüssigkeiten an einen Patienten, aufweisend einen Katheter mit einem Katheterschlauch und mit wenigstens einem Lumen, welches zwischen einem proximalen Schlauchende und einem distalen Schlauchende durch den Katheterschlauch längserstreckt ist, mehrere Pumpeinrichtungen, welche jeweils fluidleitend mit dem wenigstens einen Lumen verbunden sind, und eine mit den mehreren Pumpeinrichtungen verbundene Steuereinrichtung, welche derart zum Steuern der mehreren Pumpeinrichtungen eingerichtet ist, dass die unterschiedlichen medizinischen Flüssigkeiten mittels der mehreren Pumpeinrichtungen im Zeitmultiplex durch das wenigstens eine Lumen förderbar sind. Die Erfindung betrifft zudem einen Katheter für ein solches Infusionssystem.

Ein derartiges Infusionssystem ist beispielsweise aus der WO 2019/001879 A1 bekannt. Das bekannte Infusionssystem ist zur Bereitstellung mehrerer Infusionen für einen Patienten eingerichtet, wobei das System eine Vielzahl von Infusionsvorrichtungen zur Verabreichung einer Vielzahl von medizinischen Flüssigkeiten durch eine Infusionsleitung eines Infusionssets an den Patienten und eine Steuervorrichtung zur Steuerung der Vielzahl von Infusionsvorrichtungen umfasst. Dabei umfasst die Steuervorrichtung ein Multiplex-Modul, das so konfiguriert ist, dass es die Vielzahl medizinischer Flüssigkeiten für eine Multiplex-Verabreichung der medizinischen Flüssigkeiten durch die Infusionsleitung des Infusionssets multiplexiert (Zeitmultiplex), wobei das Multiplex-Modul ein Planungsmodul umfasst, das so konfiguriert ist, dass es mindestens zwei Pakete definiert, wobei jedes Paket mindestens eine medizinische Flüssigkeit aus der Vielzahl medizinischer Flüssigkeiten umfasst, und dass es die mindestens zwei Pakete in einer Reihenfolge für die Verabreichung der medizinischen Flüssigkeiten der mindestens zwei Pakete anordnet. Die Infusionsvorrichtungen sind über Zuleitungen mit einem Mehrfachventil verbunden, wobei das Mehrfachventil schaltbare Ventile zum Umschalten zwischen den Infusionen der verschiedenen Infusionsvorrichtungen zur Zuführung über eine einzige mit dem Patienten verbundene Infusionsleitung umfasst. Bei dem bekannten Infusionssystem kann es aufgrund der mehreren Zuleitungen zu dem Mehrfachventil zu unbeabsichtigten Durchmischungen der medizinischen Flüssigkeiten kommen. Zudem sind die Infusionsvorrichtungen aufgrund ihrer Größe an einem Gestell angeordnet, das neben dem Patienten, insbesondere neben dem Patientenbett, platziert ist. Weitere Infusionspumpenvorrichtungen sind aus den folgenden Dokumenten bekannt: DE 10 2004 047195 B4, US 2009/318902 A1 und US 2004/171985 A1.

Aufgabe der Erfindung ist es, ein Infusionssystem der eingangs genannten Art und einen Katheter für ein solches Infusionssystem bereitzustellen, die Vorteile, insbesondere hinsichtlich Funktionsweise, Aufbau und/oder Betriebszuverlässigkeit, gegenüber dem Stand der Technik bieten.

Diese Aufgabe wird für das Infusionssystem durch die Merkmale des Anspruchs 1 gelöst. Vorteilhafte Ausgestaltungen der Erfindung sind in den Unteransprüchen angegeben, deren Wortlaut hiermit durch Verweis zum Bestandteil der Beschreibung gemacht wird. Dies schließt insbesondere auch alle Ausführungsformen der Erfindung ein, die sich aus den Merkmalskombinationen ergeben, die durch die Rückbezüge in den Unteransprüchen definiert sind.

Bei dem erfindungsgemäßen Infusionssystem weist der Katheter die mehreren Pumpeinrichtungen auf, wobei die mehreren Pumpeinrichtungen jeweils als Mikropumpe gestaltet und an dem proximalen Schlauchende montiert sind. Die mehreren Mikropumpen sind vorzugsweise derart an dem proximalen Schlauchende montiert, dass ein Auslass einer jeden Mikropumpe unmittelbar in den wenigstens einen Lumen mündet. Hierdurch entsteht zwischen einer jeden Mikropumpe und dem Lumen nahezu kein Totvolumen. Die Mikropumpen sind vorzugsweise in den Katheter integriert. Bei einer anderen Ausführungsform sind die Mikropumpen zwar mittelbar, jedoch nahe am Katheterschlauch angeordnet. Das proximale Schlauchende ist - in einem angelegten Zustand des Katheters - dem Patienten abgewandt, wohingegen das distale Schlauchende dem Patienten zugewandt ist. Die Mikropumpen können dabei einer hierfür geeigneten Bauart entsprechen, z.B. der Bauart einer, insbesondere piezoelektrischen, Membranpumpe oder einer Schlauchpumpe, auch Peristaltikpumpe genannt. Je nach Bauart der Mikropumpe können die mehreren Mikropumpen mechanisch und/oder elektrisch im Zeitmultiplex angetrieben und synchronisiert werden, z.B. durch Algorithmen zur Getriebesteuerung oder Schrittmotoren oder dergleichen. Eine einzelne Mikropumpe kann eine maximale Länge von 40 mm, vorzugsweise 30 mm, und/oder eine maximale Breite von 20 mm, vorzugsweise 15 mm, und/oder eine maximale Höhe von 20 mm, vorzugsweise 15 mm, aufweisen. Zudem kann eine einzelne Mikropumpe ein maximales Bauteilgewicht von 30 g, vorzugsweise 15 g, besonders bevorzugt 5 g, aufweisen. Zusätzlich oder alternativ zu den Mikropumpen sind bei einer Ausgestaltung gesteuerte Mikroventile vorhanden. Im Betrieb des Infusionssystems werden die Mikropumpen so angesteuert, dass Dosen, insbesondere Mikrodosierungen, der verschiedenen medizinischen Flüssigkeiten nacheinander in den Katheter gepumpt werden. Dabei können zwischen den verschiedenen medizinischen Flüssigkeiten Trennflüssigkeiten eingepumpt werden, um z.B. verschiedene inkompatible Infusionen nacheinander an den Patienten zu verabreichen. Als Trennflüssigkeit kann z.B. eine Kochsalzlösung verwendet werden. Dabei versteht sich, dass das Infusionssystem grundsätzlich zur Abgabe von Fluiden, d.h. Flüssigkeiten und/oder Gasen, geeignet ist. Eine Beschränkung auf Flüssigkeiten besteht insoweit nicht. Vielmehr sind Gase ausdrücklich mit umfasst. Anstelle oder zusätzlich zu der erwähnten Kochsalzlösung kann folglich beispielsweise auch Kohlendioxid als Trennfluid verwendet werden. Die mit den mehreren Mikropumpen verbundene Steuereinrichtung kann weiter derart eingerichtet sein, dass auch mehrere Mikropumpen zeitgleich aktiv sein können. Hierdurch ist es möglich, dass medizinische Flüssigkeiten, die zueinander kompatibel und daher durchmischt werden können, zeitgleich ohne eine Trennflüssigkeit abgegeben und dem Patienten verabreicht werden können. Die Steuerung des Zeitmultiplexes kann beliebig komplex werden. Die Verbindung zwischen der Steuereinrichtung und den mehreren Mikropumpen kann drahtlos und/oder drahtgebunden sein. Der Katheterschlauch weist bei einer Ausführungsform lediglich ein einziges Lumen auf, so dass auch von einem einlumigen Katheter gesprochen werden kann. Bei weiteren Ausführungsformen weist der Katheterschlauch wenigstens zwei Lumen auf, so dass auch von einem zwei- bzw. mehrlumigen Katheter gesprochen werden kann. Hierdurch können auch verschiedene inkompatible Infusionen gleichzeitig abgegeben werden. Sofern der Katheterschlauch wenigstens zwei Lumen aufweist, ist eine dementsprechende Anzahl von Katheterzuleitungen vorhanden. Die erfindungsgemäße Lösung eignet sich insbesondere für komplexe Infusionstherapien. Durch die Verwendung von Mikropumpen, die sehr nah am Patienten platziert werden, wird das Gesamtsystem auf eine quasi-kontinuierliche Infusion mehrerer, insbesondere inkompatibler, Infusionslösungen bei gleichzeitigem Ausschluss der Risiken durch Durchmischung inkompatibler Infusionen optimiert. Zudem ermöglicht dies eine hochgenaue Dosierung, wobei ein ungewollter Bolus oder ein Rückfluss der medizinischen Flüssigkeiten vermieden werden können. Besagte Boli oder Rückflüsse könne beispielsweise durch Bewegungen langer Verbindungsschläuche zustande kommen. Zudem kann eine hochpräzise, quasi-kontinuierliche Medikamentenabgabe gewährleistet werden.

In Ausgestaltung der Erfindung sind die mehreren Mikropumpen jeweils zur Förderung mit einer Förderrate von 0,05 ml/min bis 500 ml/min, bevorzugt von 0,1 ml/min bis 300 ml/min, eingerichtet. Eine Förderrate von bis zu 300 ml/min liegt bspw. bei der sogenannten Akutdialyse vor. Katecholamine, wie z.B. Adrenalin oder Dopamin, werden verdünnt mit einer Flussrate ab 0,1 ml/min zugeführt. Bei dieser Ausgestaltung der Erfindung ist die Steuereinrichtung zum Steuern der Förderraten eingerichtet. Hierdurch ist es möglich, die Förderrate insbesondere medikamentenabhängig anzupassen. Durch hochgenaue Mikrodosierungen, die insbesondere nur bei sehr kleinem Totvolumen zwischen einer jeden Mikropumpe und dem Lumen möglich sind, können auch Medikamente mit geringer Flussrate quasikontinuierlich dem Patienten zugeführt werden. Bei weiteren Ausführungsformen können mehrere Mikropumpen miteinander kombiniert werden. Eine Kombination aus einer Mikropumpe mit relativ kleiner Förderrate und einer Mikropumpe mit relativ größer Förderrate ermöglicht es, beispielsweise bei niedrig dosierten Medikamenten die notwendige Genauigkeit und bei hoch dosierten Medikamenten die notwendige Förderleistung zu erhalten. Sofern die Mikropumpen jeweils als Peristaltikpumpe mit umlaufendem Rotor gestaltet sind, liegt die jeweilige Förderrate vorzugsweise bei 2 µl je Rotorumdrehung.

In weiterer Ausgestaltung der Erfindung sind die mehreren Mikropumpen jeweils zur intermittierenden Förderung mit einer Taktung zwischen 1 s und 100 s, bevorzugt zwischen 5 s und 20 s, eingerichtet. Die Steuereinrichtung ist zum Steuern der Taktungen eingerichtet. Hierdurch ist es möglich, die Taktung insbesondere medikamentenabhängig anzupassen. Bei kritischen Medikamenten, wie z.B. Adrenalin, kann die Steuereinrichtung derart gesteuert werden, dass eine diskret getaktete Medikamentenabgabe vergleichbar mit einer kontinuierlichen Medikamentenabgabe wirkt. Die Mikropumpen werden jeweils entsprechend der vorgegebenen Taktung abwechselnd aktiviert und deaktiviert, um eine Mikrodosierung durch den Katheter zu fördern. Bei einer weiteren Ausgestaltung der Erfindung kann die Taktung zur intermittierenden Förderung von, insbesondere nicht kritischen, Medikamenten mehr als 100 s betragen.

In weiterer Ausgestaltung der Erfindung ist die Steuereinrichtung zum Steuern der Förderraten und/oder der Taktungen in Abhängigkeit einer vorgegebenen Gesamtförderrate eingerichtet. Die Gesamtförderrate setzt sich aus den vorliegenden Förderraten der einzelnen Mikropumpen zusammen. Die Steuereinrichtung steuert demnach die Förderrate und/oder die Taktung jeder einzelnen Mikropumpe in Abhängigkeit der vorgegebenen Gesamtförderrate. Diese Ausgestaltung erlaubt, vereinfacht ausgedrückt, eine quasi-konstante Gesamtförderrate des Infusionssystems, d.h. die diskreten Abgaben der einzelnen Infusionen wirken aufgrund des sehr geringen zeitlichen Abstands wie eine kontinuierliche Medikamentenabgabe auf den Patienten.

In weiterer Ausgestaltung der Erfindung umfassen die mehreren Mikropumpen eine erste Mikropumpe zum Fördern einer ersten medizinischen Flüssigkeit, eine zweite Mikropumpe zum Fördern einer zweiten medizinischen Flüssigkeit und eine dritte Mikropumpe zum Fördern einer dritten medizinischen Flüssigkeit. Dabei ist die Steuereinrichtung derart zum Steuern der ersten, zweiten und dritten Mikropumpe eingerichtet, dass erste Dosen der ersten medizinischen Flüssigkeit und zweite Dosen der zweiten medizinischen Flüssigkeit mittels der dritten medizinischen Flüssigkeit örtlich getrennt im Zeitmultiplex durch das wenigstens eine Lumen förderbar sind. Die dritte medizinische Flüssigkeit kann vorzugsweise eine Trennflüssigkeit, wie z.B. eine Kochsalzlösung, sein. Hierdurch ist es möglich, medizinische Flüssigkeiten, die zueinander inkompatibel sind, durch die Trennflüssigkeit örtlich zu trennen und trotzdem in einem einzigen Lumen zu fördern. Die Trennflüssigkeit kann kontinuierlich oder diskret getaktet gesteuert gefördert werden. Bei einer kontinuierlichen Förderung der Trennflüssigkeit werden die medizinischen Flüssigkeiten der Trennflüssigkeit zugeführt und damit durchmischt. Hierbei wird die Trennflüssigkeit zeitweise zu einer Art Trägerflüssigkeit. Die inkompatiblen Flüssigkeiten bleiben dadurch weiterhin voneinander getrennt.

In weiterer Ausgestaltung der Erfindung ist wenigstens die erste Mikropumpe zur Umkehr einer Förderrichtung eingerichtet. Die Steuereinrichtung ist derart zum Steuern der ersten Mikropumpe eingerichtet, dass eine in das wenigstens eine Lumen abgegebene erste Dosis der ersten medizinischen Flüssigkeit mittels einer Umkehr der Förderrichtung teilweise aus dem wenigstens einen Lumen ansaugbar ist. Durch das Ansaugen der ersten medizinischen Flüssigkeit mittels der ersten Mikropumpe kann eine weitere, insbesondere unbeabsichtigte, Abgabe der ersten medizinischen Flüssigkeit in das wenigstens eine Lumen zuverlässig und/oder eine Durchmischung der ersten medizinischen Flüssigkeit mit einer weiteren medizinischen Flüssigkeit verhindert werden. Hierdurch ist es möglich, eine Durchmischung der, insbesondere inkompatiblen, medizinischen Flüssigkeiten bereits bei der Abgabe in das Lumen zu vermeiden. Bei dem Ansaugen der ersten medizinischen Flüssigkeit kann zusätzlich eine Trennflüssigkeit oder dergleichen angesaugt werden. Dies wirkt einer unbeabsichtigten Durchmischung inkompatibler Flüssigkeiten zusätzlich entgegen.

In weiterer Ausgestaltung der Erfindung weist der Katheterschlauch wenigstens ein weiteres Lumen auf, wobei das Lumen eine hydrophobe Oberflächenbeschaffenheit und das weitere Lumen eine lipophobe Oberflächenbeschaffenheit aufweist oder umgekehrt. Die Oberflächenbeschaffenheit eines Lumens wird durch eine Oberflächenbeschaffenheit einer dieses Lumen begrenzenden Innenseite einer Schlauchwandung des Katheterschlauchs definiert. Bei einer hydrophoben Oberflächenbeschaffenheit weist die Oberfläche in der Art des Lotus-Effekts eine geringe Benetzbarkeit für wasserbasierte Flüssigkeiten auf, so dass bei wasserbasierten medizinischen Flüssigkeiten das Fließverhalten und/oder die Förderrate aufgrund einer minimierten Haftung an der Innenseite des Katheterschlauchs begünstigt wird. Im Gegensatz dazu weist bei einer lipophoben Oberflächenbeschaffenheit die Oberfläche in der Art des Lotus-Effekts eine geringe Benetzbarkeit für fettbasierte Flüssigkeiten auf, so dass bei fettbasierten medizinischen Flüssigkeiten das Fließverhalten und/oder die Förderrate aufgrund einer minimierten Haftung an der Innenseite des Katheterschlauchs begünstigt wird. Bei dieser Ausgestaltung der Erfindung wird bei entsprechender Anwendung durch die Oberflächenbeschaffenheiten der Lumen eine rückstandsfreie Förderung medizinischer Flüssigkeiten begünstigt, so dass wiederum ein unbeabsichtigtes Durchmischen der medizinischen Flüssigkeiten verhindert werden kann.

In weiterer Ausgestaltung der Erfindung weist der Katheter einen Spritzenadapter auf. Bei dieser Ausgestaltung der Erfindung ist es möglich, z.B. bei Notfällen, dem Katheter und damit dem Patienten schnell und einfach eine bestimmte medizinische Flüssigkeit über den Spritzenadapter zuzuführen. Der Spritzenadapter ist vorzugsweise konform zu einem Luer-Konnektor, einem NRFit-Konnektor, einem Non-Luer-Konnektor oder dergleichen ausgebildet. Der Spritzenadapter ist fluidleitend mit dem wenigstens einen Lumen verbunden und vorzugsweise unlösbar am Katheterschlauch montiert, vorzugsweise an seinem proximalen Schlauchende.

In weiterer Ausgestaltung ist der Katheter als Einwegprodukt zur einmaligen Verwendung eingerichtet. Dabei sind die mehreren Mikropumpen vorzugsweise unlösbar an dem proximalen Schlauchende des Katheterschlauchs montiert. Die Steuereinrichtung ist hingegen zur mehrfachen Verwendung eingerichtet.

Der erfindungsgemäße Katheter für ein erfindungsgemäßes Infusionssystem umfasst einen Katheterschlauch und wenigstens ein Lumen, welches zwischen einem proximalen Schlauchende und einem distalen Schlauchende durch den Katheterschlauch längserstreckt ist. Der Katheter umfasst weiter mehrere Pumpeinrichtungen, welche jeweils als Mikropumpe gestaltet und an dem proximalen Schlauchende montiert sind. Der erfindungsgemäße Katheter kann z.B. als zentralvenöser Katheter verwendet werden, der über eine Vene der oberen Körperhälfte des Patienten in das Venensystem eingeführt wird und dessen Ende in der oberen oder unteren Hohlvene vor dem rechten Vorhof des Herzens liegt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch stark vereinfachter Darstellung eine Ausführungsform eines erfindungsgemäßen Infusionssystems mit einer Ausführungsform eines erfindungsgemäßen Katheters und einer Steuereinrichtung,
- Fig. 2: in schematisch stark vereinfachter Schnittdarstellung einen einlumigen Katheterschlauch des Katheters nach Fig. 1, bei einer Förderung mehrerer medizinischer Flüssigkeiten entlang einer Förderrichtung,
- Fig. 3: in schematisch stark vereinfachter Schnittdarstellung einen zweilumigen Katheterschlauch als Variante zu dem Katheterschlauch nach Fig. 2, und
- Fig. 4A bis 4D: je eine schematisch stark vereinfachte Schnittdarstellung zur Veranschaulichung aufeinanderfolgender Schritte zur Funktionsweise des Infusionssystems nach Fig. 1.

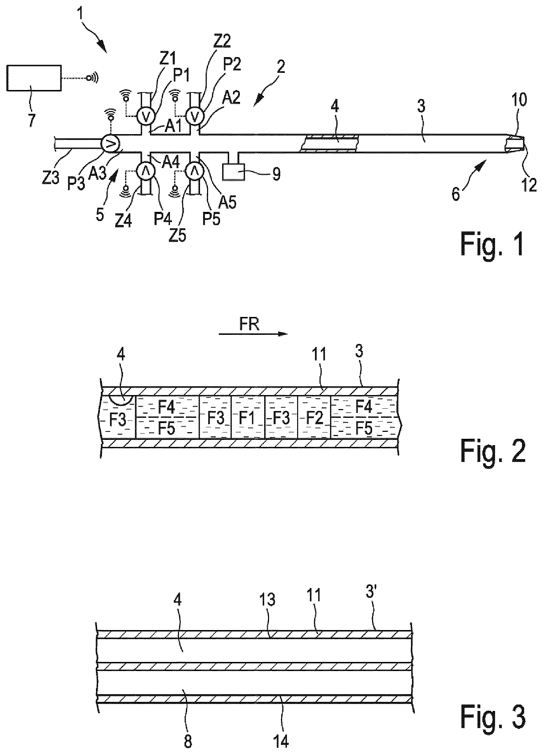

Ein Infusionssystem 1 nach Fig. 1 ist zum Abgeben unterschiedlicher medizinischen Flüssigkeiten F1 bis F5 an einen Patienten eingerichtet. Das Infusionssystem 1 ist schematisch stark vereinfacht dargestellt und weist einen Katheter 2 und eine Steuereinrichtung 7 auf. Der Katheter 2 ist bei der Ausführungsform gemäß der Fig. 1 einlumig ausgebildet und weist einen Katheterschlauch 3 mit einem (einzigen) Lumen 4 auf. Das Lumen 4 ist zwischen einem proximalen Schlauchende 5 und einem distalen Schlauchende 6 durch den Katheterschlauch 3 längserstreckt.

Bei alternativen Ausführungen ist der Katheter in Form eines zweilumigen Katheter ausgebildet, wie in der Fig. 3 ersichtlich ist. Weiter alternativ kann der Katheter mehr als zwei Lumen aufweisen. Bei mehrlumigen Kathetern können auch verschiedene inkompatible Infusionen gleichzeitig an den Patienten abgegeben werden.

Der Katheter 2 weist weiter mehrere Pumpeinrichtungen auf, welche jeweils fluidleitend mit dem Lumen 4 verbunden sind, wie in der Fig. 1 ersichtlich ist. In dem gezeigten Ausführungsbeispiel weist der Katheter 2 fünf Pumpeinrichtungen auf.

Die genaue Anzahl der mehreren Pumpeinrichtungen ist nicht wesentlich für die zugrunde liegende Erfindung. Bei einer zeichnerisch nicht dargestellten Ausführungsform weist der Katheter 2 zwei, drei, vier oder mehr als fünf, mehr als 10 oder gar mehr als 20 Pumpeinrichtungen auf.

Vorliegend sind die mehreren Pumpeinrichtungen jeweils als Mikropumpe P1 bis P5 gestaltet und an dem proximalen Schlauchende 5 montiert. Bei den gezeigten Ausführungsformen sind die mehreren Mikropumpen P1 bis P5 derart an dem proximalen Schlauchende 5 montiert, dass ein Auslass A1 bis A5 einer jeden Mikropumpe P1 bis P5 unmittelbar in das Lumen 4 mündet, wie in der Fig. 1 ersichtlich ist. Vorliegend sind die Mikropumpen P1, P2, P4 und P5 in radialer Richtung zu einer Schlauchwandung 11 des Katheterschlauchs 3 angeordnet, wohingegen die Mikropumpe P3 in axialer Richtung zu der Schlauchwandung 11 des Katheterschlauchs 3 am proximalen Schlauchende 5 angeordnet ist. Bei einer weiteren Ausführungsform sind alternativ alle Mikropumpen bis in radialer Richtung zu der Schlauchwandung des Katheterschlauchs angeordnet.

Der Katheterschlauch 3 weist in dem gezeigten Ausführungsbeispiel gemäß der Fig. 1 am distalen Schlauchende 6 eine distale Katheterspitze 10 auf. Vorliegend ist die distale Katheterspitze 10 integraler Bestandteil des Katheterschlauchs 3 und insoweit durch dessen distales Schlauchende 6 gebildet. Bei einer zeichnerisch nicht dargestellten Ausführungsform ist distale Katheterspitze 10 in Form eines separaten Bauteils gestaltet und fest mit dem distalen Schlauchende 6 des Katheterschlauchs 3 zusammengefügt. Das eine Lumen 4 weist in der gezeigten Ausführung eine distale Austrittsöffnung 12 im Bereich der Katheterspitze 10 auf, wie in der Fig. 1 ersichtlich ist.

Der Katheterschlauch 3 ist vorliegend auf eine dem Fachmann bekannte Weise aus einem biegeflexiblen und für medizinische Anwendungen geeigneten Kunststoffmaterial gefertigt.

In den Figuren sind die Mikropumpen P1 bis P5 lediglich schematisch stark vereinfacht dargestellt. Die Mikropumpen P1 bis P5 können z.B. nach Bauart einer Membranpumpe oder einer Schlauchpumpe ausgebildet sein und je nach Bedarf und Anwendungsfall mechanisch und/oder elektrisch angetrieben und synchronisiert werden.

Die mehreren Mikropumpen P1 bis P5 weisen gemäß der Fig. 1 einlassseitig Zuleitungen Z1 bis Z5 auf, die in grundsätzlich bekannter Weise fluidleitend mit in den Figuren nicht dargestellten Infusionsbeuteln oder dergleichen fluidleitend verbunden sind.

Die Steuereinrichtung 7 ist mit den mehreren Mikropumpen P1 bis P5 verbunden. Bei den gezeigten Ausführungsformen ist die Steuereinrichtung 7 drahtlos mit den mehreren Mikropumpen P1 bis P5 verbunden. Bei einer zeichnerisch nicht dargestellten Ausführungsform ist die Steuereinrichtung 7 mit den mehreren Mikropumpen P1 bis P5 drahtgebunden verbunden. Die Steuereinrichtung 7 ist derart zum Steuern der mehreren Mikropumpen P1 bis P5 eingerichtet, dass die unterschiedlichen medizinischen Flüssigkeiten F1 bis F5 mittels der mehreren Mikropumpen P1 bis P5 im Zeitmultiplex durch das Lumen 4 in eine Förderrichtung FR förderbar sind, wie in der Fig. 2 ersichtlich ist. Dabei werden die Mikropumpen P1 bis P5 so angesteuert, dass vorgegebene Dosen, insbesondere Mikrodosierungen, der unterschiedlichen medizinischen Flüssigkeiten F1 bis F5 in vorgegebenen Taktungen in den Katheter 2 gepumpt werden. Bei den gezeigten Ausführungsformen ist die Steuereinrichtung 7 derart eingerichtet, dass die mehreren Mikropumpen P1 bis P5 auch zeitgleich aktiv sein können. Hierdurch ist es möglich, dass mehrere medizinische Flüssigkeiten, die zueinander kompatibel sind und daher durchmischt werden können, selbst bei einem einlumigen Katheter zeitgleich abgegeben werden können, wie dies beispielhaft in der Fig. 2 mit den medizinischen Flüssigkeiten F4 und F5 dargestellt ist. Bei einer Ausführungsform mit einem zweilumigen Katheter können dadurch auch unterschiedliche, zueinander inkompatible Infusionen gleichzeitig in jeweils einem Lumen an den Patienten abgegeben werden. Im Detail zeigt die Fig. 2 eine schematisch stark vereinfachte Schnittdarstellung des einlumigen Katheterschlauchs 3 gemäß der Fig. 1. Im Katheterschlauch 3 sind einzelne Dosen, insbesondere Mikrodosierungen, der mehreren medizinischen Flüssigkeiten F1 bis F5 mit einer Förderrichtung FR dargestellt. In dem gezeigten Ausführungsbeispiel werden die einzelnen Dosen der medizinischen Flüssigkeiten F1 bis F3 nacheinander entlang der Förderrichtung FR gefördert, wohingegen die einzelnen Dosen der medizinischen Flüssigkeiten F4 und F5 gleichzeitig, insbesondere miteinander durchmischt, gefördert werden.

Bei den gezeigten Ausführungsformen sind die mehreren Mikropumpen P1 bis P5 jeweils zur Förderung mit einer Förderrate von 0,05 ml/min bis 500 ml/min, vorzugsweise von 0,1 ml/min bis 300 ml/min, eingerichtet. Vorliegend ist die Steuereinrichtung 7 zum Steuern der Förderraten jeder einzelnen Mikropumpen P1 bis P5 eingerichtet. Bei dieser Ausgestaltung der Erfindung ist vorzugsweise die Förderrate einer jeden Mikropumpe P1 bis P5 durch die Steuereinrichtung 7 insbesondere medikamentenabhängig einstellbar.

Bei den gezeigten Ausführungsformen sind die mehreren Mikropumpen P1 bis P5 jeweils zur intermittierenden Förderung mit einer Taktung zwischen 1s und 100s, bevorzugt zwischen 5s und 20s, eingerichtet. Vorliegend ist die Steuereinrichtung 7 zum Steuern der Taktungen eingerichtet. Bei dieser Ausgestaltung der Erfindung ist vorzugsweise die Taktung einer jeden Mikropumpe P1 bis P5 durch die Steuereinrichtung 7 insbesondere medikamentenabhängig einstellbar. Bei weiteren Ausführungsformen ist die Förderrate der Mikropumpen P1 bis P5 steuerbar ausgelegt, d.h. die Fördermenge der Mikropumpen P1 bis P5 ist nicht nur von der Taktlänge, sondern auch von dem Bedarf abhängig.

Bei den gezeigten Ausführungsformen ist die Steuereinrichtung 7 zum Steuern der Förderraten und/oder der Taktungen in Abhängigkeit einer vorgegebenen Gesamtförderrate eingerichtet. Vorliegend setzt sich die Gesamtförderrate aus den Förderraten der einzelnen Mikropumpen P1 bis P5 zusammen. Die Steuereinrichtung 7 steuert vorzugsweise die Förderrate und/oder die Taktung jeder einzelnen Mikropumpe P1 bis P5 in Abhängigkeit der vorgegebenen Gesamtförderrate.

Bei den gezeigten Ausführungsformen umfassen die mehreren Mikropumpen P1 bis P5 eine erste Mikropumpe P1 zum Fördern einer ersten medizinischen Flüssigkeit F1, eine zweite Mikropumpe P2 zum Fördern einer zweiten medizinischen Flüssigkeit F2 und eine dritte Mikropumpe P3 zum Fördern einer dritten medizinischen Flüssigkeit F3. Vorliegend ist die Steuereinrichtung 7 derart zum Steuern der ersten, zweiten und dritten Mikropumpe P1, P2, P3 eingerichtet, dass erste Dosen der ersten medizinischen Flüssigkeit F1 und zweite Dosen der zweiten medizinischen Flüssigkeit F2 mittels der dritten medizinischen Flüssigkeit F3 örtlich getrennt im Zeitmultiplex durch das wenigstens eine Lumen 4 nacheinander förderbar sind, wie dies insbesondere in der Fig. 2 ersichtlich ist. Bei der dritten medizinischen Flüssigkeit F3 handelt es sich vorzugsweise um eine Trennflüssigkeit, wie z.B. eine Kochsalzlösung. Bei dem gezeigten Ausführungsbeispiel gemäß der Fig. 2 wird die Trennflüssigkeit in Form der dritten medizinische Flüssigkeit F3 zwischen der ersten medizinischen Flüssigkeit F1 und der zweiten medizinischen Flüssigkeit F2 eingepumpt und trennt diese dadurch örtlich voneinander. Die dritte medizinische Flüssigkeit F3 ist vorliegend zu der ersten medizinischen Flüssigkeit F1 und der zweiten medizinischen Flüssigkeit F2 kompatibel.

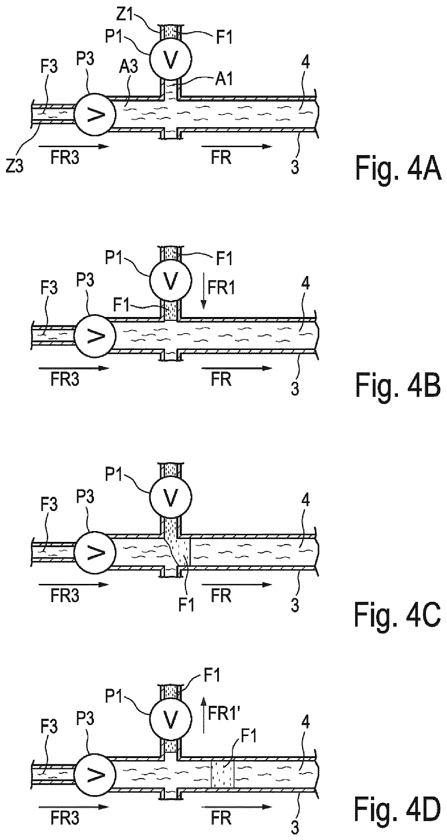

Bei den gezeigten Ausführungsformen ist wenigstens die erste Mikropumpe P1 zur Umkehr ihrer Förderrichtung FR1 eingerichtet. Vorliegend ist die Steuereinrichtung 7 derart zum Steuern der ersten Mikropumpe P1 eingerichtet, dass eine in das wenigstens eine Lumen 4 geförderte erste Dosis der ersten medizinischen Flüssigkeit F1 mittels einer Umkehr der Förderrichtung FR1 teilweise aus dem wenigstens einen Lumen 4 ansaugbar ist, wie dies in den Fig. 4A bis 4D dargestellt ist.

In den Fig. 4A bis 4D ist je eine schematisch stark vereinfachte Schnittdarstellung zur Veranschaulichung aufeinanderfolgender Schritte zur Funktionsweise der Ausführungsform des Infusionssystems 1 nach Fig. 1 dargestellt. Vorliegend wird beispielhaft die Funktionsweise anhand der Mikropumpen P1 und P3 beschrieben. Die Mikropumpen P2, P4 und P5 sind in den Fig. 4A bis 4D nicht gesondert dargestellt. Wie bereits angesprochen ist die erste Mikropumpe P1 zum Fördern der ersten medizinischen Flüssigkeit F1 und die dritte Mikropumpe P3 zum Fördern der dritten medizinischen Flüssigkeit F3 eingerichtet, wobei vorliegend die dritte medizinische Flüssigkeit F3 eine Trennflüssigkeit ist. In dem gezeigten Ausführungsbeispiel wird die dritte Mikropumpe P3 derart angesteuert, dass eine kontinuierliche Förderung der dritten medizinischen Flüssigkeit F3 in einer Förderrichtung FR3 in das Lumen 4 erfolgt. Die erste Mikropumpe P1 ist vorliegend zeitlich gesteuert. In der Fig. 4A ist die erste Mikropumpe P1 deaktiviert, so dass diese eine Förderrate von 0 ml/min aufweist. Wird die erste Mikropumpe P1 kurzzeitig aktiviert, dann fördert diese eine Dosis der ersten medizinischen Flüssigkeit F1 entlang ihrer Förderrichtung FR1 in das Lumen 4, wie in den Fig. 4B ersichtlich ist. In der Fig. 4C ist die erste Mikropumpe P1 erneut deaktiviert. Die dritte medizinische Flüssigkeit F3 fungiert als Trägerflüssigkeit und nimmt zumindest einen Teil der geförderten Dosis der ersten medizinischen Flüssigkeit F1 mit entlang der Förderrichtung FR, wie in der Fig. 4D ersichtlich ist. In der Fig. 4D wird die erste Mikropumpe P1 derart angesteuert, dass sich deren Förderrichtung FR1 in eine dazu entgegengesetzte Förderrichtung FR1' umkehrt. Dadurch wird ein noch nicht in das Lumen 4 geförderter, restlicher Teil der Dosis der ersten medizinischen Flüssigkeit F1 und vorzugsweise eine geringe Dosis der dritten medizinischen Flüssigkeit F3, d.h. vorliegend der Trennflüssigkeit, in die entgegengesetzte Förderrichtung FR1', eine Richtung weg von dem Lumen 4, angesaugt. Hierdurch kann eine weitere, insbesondere unbeabsichtigte, Mitnahme der ersten medizinischen Flüssigkeit F1 durch die dritte medizinische Flüssigkeit F3 oder durch eine weitere, mittels einer der nicht gesondert gezeigten Mikropumpen P2, P4, P5 geförderten, medizinische Flüssigkeit vermieden werden. Folglich wird einer Durchmischung von inkompatiblen medizinischen Flüssigkeiten besonders effektiv entgegengewirkt

Bei der Variante gemäß der Fig. 3 weist der Katheterschlauch 3' wenigstens ein weiteres Lumen 8 auf, wobei das Lumen 4 eine hydrophobe Oberflächenbeschaffenheit und das weitere Lumen 8 eine lipophobe Oberflächenbeschaffenheit aufweist oder umgekehrt. Die Oberflächenbeschaffenheit des Lumens 4 ist durch eine Oberflächenbeschaffenheit einer dieses Lumen 4 begrenzenden Innenseite 13 der Schlauchwandung 11 des Katheterschlauchs 3' definiert. Durch die hydrophobe Oberflächenbeschaffenheit des Lumens 4 weist dieses in der Art des Lotus-Effekts eine geringe Benetzbarkeit für wasserbasierte Flüssigkeiten auf, so dass bei wasserbasierten medizinischen Flüssigkeiten das Fließverhalten und/oder die Förderrate aufgrund einer minimierten Haftung an der Innenseite 13 der Schlauchwandung 11 begünstigt wird. Die Oberflächenbeschaffenheit des weiteren Lumens 8 ist durch eine Oberflächenbeschaffenheit einer dieses weitere Lumen 8 begrenzenden Innenseite 14 der Schlauchwandung 11 des Katheterschlauchs 3 definiert. Durch die lipophobe Oberflächenbeschaffenheit des weiteren Lumens 8 weist dieses in der Art des Lotus-Effekts eine geringe Benetzbarkeit für fettbasierte Flüssigkeiten auf, so dass bei fettbasierten medizinischen Flüssigkeiten das Fließverhalten und/oder die Förderrate aufgrund einer minimierten Haftung an der Innenseite 14 der Schlauchwandung 11 begünstigt wird. Vorliegend wird bei entsprechender Anwendung durch die unterschiedlichen Oberflächenbeschaffenheiten der Lumen 4, 8 eine rückstandsfreie Förderung medizinischer Flüssigkeiten F1 bis F5 begünstigt, so dass wiederum ein unbeabsichtigtes Durchmischen der medizinischen Flüssigkeiten F1 bis F5 verhindert werden kann.

Bei den gezeigten Ausführungsformen weist der Katheter 2 einen Spritzenadapter 9 auf, der in Fig. 1 schematisch stark vereinfacht als Block dargestellt ist. Der Spritzenadapter 9 fungiert als eine Art Fluidkonnektor und ist mit dem wenigstens einen Lumen 4 fluidleitend verbunden. Bei unterschiedlichen Ausführungsformen kann der Spritzenadapter 9 unterschiedlich gestaltet sein und kann beispielsweise ein Luer-Konnektor, ein NRFit-Konnektor, ein Non-Luer-Konnektor oder dergleichen sein. Hierdurch ist es möglich, z.B. bei Notfällen, dem Katheter 2 und damit dem Patienten schnell und einfach eine bestimmte medizinische Flüssigkeit über den Spritzenadapter 9 zuzuführen. Vorliegend ist der Spritzenadapter 9 am proximalen Schlauchende 5 angeordnet.

Bei weiteren Ausführungsformen kann der Spritzenadapter 9 weiter in Richtung des distalen Schlauchendes 6 angeordnet sein.

Bei den gezeigten Ausführungsformen ist der Katheter 2 als Einwegprodukt zur einmaligen Verwendung eingerichtet. Die Steuereinrichtung 7 ist vorzugsweise zur mehrfachen Verwendung eingerichtet.

Vorliegend sind die mehreren Mikropumpen P1 bis P5 unlösbar an dem proximalen Schlauchende 5 des Katheterschlauchs 3 montiert.

## Patentansprüche

1. Infusionssystem (1) zum Abgeben unterschiedlicher medizinischen Flüssigkeiten (F1 bis F5) an einen Patienten, aufweisend
einen Katheter (2) mit einem Katheterschlauch (3) und mit wenigstens einem Lumen (4), welches zwischen einem proximalen Schlauchende (5) und einem distalen Schlauchende (6) durch den Katheterschlauch (3) längserstreckt ist,
mehrere Pumpeinrichtungen, welche jeweils fluidleitend mit dem wenigstens einen Lumen (4) verbunden sind, und
eine mit den mehreren Pumpeinrichtungen verbundene Steuereinrichtung (7), welche derart zum Steuern der mehreren Pumpeinrichtungen eingerichtet ist, dass die unterschiedlichen medizinischen Flüssigkeiten (F1 bis F5) mittels der mehreren Pumpeinrichtungen im Zeitmultiplex durch das wenigstens eine Lumen (4) förderbar sind,
wobei der Katheter (2) die mehreren Pumpeinrichtungen aufweist,
wobei die mehreren Pumpeinrichtungen jeweils als Mikropumpe (P1 bis P5) gestaltet und an dem proximalen Schlauchende (5) montiert sind,
wobei die mehreren Mikropumpen (P1 bis P5) eine erste Mikropumpe (P1) zum Fördern einer ersten medizinischen Flüssigkeit (F1), eine zweite Mikropumpe (P2) zum Fördern einer zweiten medizinischen Flüssigkeit (F2) und eine dritte Mikropumpe (P3) zum Fördern einer dritten medizinischen Flüssigkeit (F3) umfassen,
wobei die Steuereinrichtung (7) derart zum Steuern der ersten Mikropumpe (P1), der zweiten Mikropumpe (P2) und der dritten Mikropumpe (P3) eingerichtet ist, dass erste Dosen der ersten medizinischen Flüssigkeit (F1) und zweite Dosen der zweiten medizinischen Flüssigkeit (F2) mittels der dritten medizinischen Flüssigkeit (F3) örtlich getrennt im Zeitmultiplex durch das wenigstens eine Lumen (4) förderbar sind,
**dadurch gekennzeichnet, dass** die erste Mikropumpe (P1) zur Umkehr einer Förderrichtung (FR1) eingerichtet ist, und die Steuereinrichtung (7) derart zum Steuern der ersten Mikropumpe (P1) eingerichtet ist, dass eine in das wenigstens eine Lumen (4) abgegebene erste Dosis der ersten medizinischen Flüssigkeit (F1) mittels einer Umkehr der Förderrichtung (FR1) teilweise aus dem wenigstens einen Lumen (4) ansaugbar ist.

2. Infusionssystem (1) nach Anspruch 1, **dadurch gekennzeichnet, dass** die mehreren Mikropumpen (P1 bis P5) jeweils zur Förderung mit einer Förderrate von 0,05 ml/min bis 500 ml/min, bevorzugt von 0,1 ml/min bis 300 ml/min, eingerichtet sind, und dass die Steuereinrichtung (7) zum Steuern der Förderraten eingerichtet ist.

3. Infusionssystem (1) nach Anspruch 1 oder 2, **dadurch gekennzeichnet, dass** die mehreren Mikropumpen (P1 bis P5) jeweils zur intermittierenden Förderung mit einer Taktung zwischen 1 s und 100 s, bevorzugt zwischen 5 s und 20 s, eingerichtet sind, und die Steuereinrichtung (7) zum Steuern der Taktungen eingerichtet ist.

4. Infusionssystem (1) nach Anspruch 2 oder 3, **dadurch gekennzeichnet, dass** die Steuereinrichtung (7) zum Steuern der Förderraten und/oder der Taktungen in Abhängigkeit einer vorgegebenen Gesamtförderrate eingerichtet ist.

5. Infusionssystem (1) nach einem der vorhergehenden Ansprüche, **dadurch gekennzeichnet, dass** der Katheterschlauch (3) wenigstens ein weiteres Lumen (8) aufweist, wobei das Lumen (4) eine hydrophobe Oberflächenbeschaffenheit und das weitere Lumen (8) eine lipophobe Oberflächenbeschaffenheit aufweist oder umgekehrt.

6. Infusionssystem (1) nach einem der vorhergehenden Ansprüche, **dadurch gekennzeichnet, dass** der Katheter (2) einen Spritzenadapter (9) aufweist.

7. Infusionssystem (1) nach einem der vorhergehenden Ansprüche, **dadurch gekennzeichnet, dass** der Katheter (2) als Einwegprodukt zur einmaligen Verwendung eingerichtet ist, und die Steuereinrichtung (7) zur mehrfachen Verwendung eingerichtet ist.

## Claims

1. An infusion system (1) for providing different medical fluids (F1 to F5) to a patient, having
a catheter (2) with a catheter tube (3) and at least one lumen (4), which extends longitudinally through the catheter tube (3) between a proximal tube end (5) and a distal tube end (6),
a plurality of pump devices, which are each connected to the at least one lumen (4) so as to convey fluid, and
a control device (7), which is connected to the plurality of pump devices and is adapted to control the plurality of pump devices in such a way that the different medical fluids (F1 to F5) can be delivered through the at least one lumen (4) by means of the plurality of pump devices in time division multiplex,
wherein the catheter (2) comprises the plurality of pump devices,
wherein the plurality of pump devices each being configured as a micropump (P1 to P5) and being mounted at the proximal tube end (5),
wherein the plurality of micropumps (P1 to P5) comprise a first micropump (P1) for delivering a first medical fluid (F1), a second micropump (P2) for delivering a second medical fluid (F2) and a third micropump (P3) for delivering a third medical fluid (F3),
wherein the control device (7) being adapted to control the first micropump (P1), the second micropump (P2) and the third micropump (P3) in such a way that first doses of the first medical fluid (F1) and second doses of the second medical fluid (F2) can be delivered through the at least one lumen (4) in time division multiplex while being physically separated by means of the third medical fluid (F3)
**characterized in that** the first micropump (P1) is adapted to reverse a delivery direction (FR1), and the control device (7) is adapted to control the first micropump (P1) in such a way that a first dose of the first medical fluid (F1), provided into the at least one lumen (4), can be partially aspirated from the at least one lumen (4) by means of a reversal of the delivery direction (FR1).

2. The infusion system (1) according to Claim 1, **characterized in that** the plurality of micropumps (P1 to P5) are each adapted to deliver with a delivery rate of from 0.05 ml/min to 500 ml/min, preferably from 0.1 ml/min to 300 ml/min, and **in that** the control device (7) is adapted to control the delivery rates.

3. The infusion system (1) according to Claim 1 or 2, **characterized in that** the plurality of micropumps (P1 to P5) are each adapted for intermittent delivery with a cycle time of between 1 s and 100 s, preferably between 5 s and 20 s, and **in that** the control device (7) is adapted to control the cycle times.

4. The infusion system (1) according to Claim 2 or 3, **characterized in that** the control device (7) is adapted to control the delivery rates and/or the cycle times as a function of a predetermined overall delivery rate.

5. The infusion system (1) according to any one of the preceding claims, **characterized in that** the catheter tube (3) has at least one further lumen (8), the lumen (4) having hydrophobic surface properties and the further lumen (8) having lipophobic surface properties, or vice versa.

6. The infusion system (1) according to any one of the preceding claims, **characterized in that** the catheter (2) has a syringe adapter (9).

7. The infusion system (1) according to any one of the preceding claims, **characterized in that** the catheter (2) is adapted as a disposable product for single use, and the control device (7) is adapted for multiple use.

## Revendications

1. Système de perfusion (1) destiné à délivrer différents liquides médicaux (F1 à F5) à un patient, possédant
un cathéter (2) comprenant un tuyau de cathéter (3) et comprenant au moins une lumière (4), laquelle s'étend longitudinalement entre une extrémité de tuyau proximale (5) et une extrémité de tuyau distale (6) à travers le tuyau de cathéter (3),
plusieurs dispositifs de pompage, lesquels sont respectivement reliés de manière fluidique à l'au moins une lumière (4), et
un dispositif de commande (7) relié aux plusieurs dispositifs de pompage, lequel est conçu pour commander les plusieurs dispositifs de pompage de telle sorte que les différents liquides médicaux (F1 à F5) puissent être transportés en multiplexage temporel à travers l'au moins une lumière (4) au moyen des plusieurs dispositifs de pompage,
le cathéter (2) possédant les plusieurs dispositifs de pompage,
les plusieurs dispositifs de pompage étant respectivement réalisés sous la forme d'une micropompe (P1 à P5) et étant montés à l'extrémité de tuyau proximale (5),
les plusieurs micropompes (P1 à P5) comprenant une première micropompe (P1) destinée à transporter un premier liquide médical (F1), une deuxième micropompe (P2) destinée à transporter un deuxième liquide médical (F2) et une troisième micropompe (P3) destinée à transporter un troisième liquide médical (F3),
le dispositif de commande (7) étant conçu pour commander la première micropompe (P1), la deuxième micropompe (P2) et la troisième micropompe (P3) de telle sorte que les premières doses du premier liquide médical (F1) et les deuxièmes doses du deuxième liquide médical (F2) peuvent être transportées au moyen du troisième liquide médical (F3) localement séparément en multiplexage temporel à travers l'au moins une lumière (4),
**caractérisé en ce que** la première micropompe (P1) est conçue pour inverser un sens de transport (FR1) et le dispositif de commande (7) est conçu pour commander la première micropompe (P1) de telle sorte qu'une première dose du premier liquide médical (F1) délivrée dans l'au moins une lumière (4) peut être partiellement aspirée hors de l'au moins une lumière (4) au moyen d'une inversion du sens de transport (FR1).

2. Système de perfusion (1) selon la revendication 1, **caractérisé en ce que** les plusieurs micropompes (P1 à P5) sont respectivement conçues pour le transport à un débit de transport de 0,05 ml/min à 500 ml/min, de préférence de 0,1 ml/min à 300 ml/min, et **en ce que** le dispositif de commande (7) est conçu pour commander les débits de transport.

3. Système de perfusion (1) selon la revendication 1 ou 2, **caractérisé en ce que** les plusieurs micropompes (P1 à P5) sont respectivement conçues pour le transport intermittent avec une cadence comprise entre 1 s et 100 s, de préférence entre 5 s et 20 s, et **en ce que** le dispositif de commande (7) est conçu pour commander les cadences.

4. Système de perfusion (1) selon la revendication 2 ou 3, **caractérisé en ce que** le dispositif de commande (7) est conçu pour commander les débits de transport et/ou les cadences en fonction d'un débit de transport global prédéfini.

5. Système de perfusion (1) selon l'une des revendications précédentes, **caractérisé en ce que** le tuyau de cathéter (3) possède au moins une lumière supplémentaire (8), la lumière (4) présentant une texture de surface hydrophobe et la lumière supplémentaire (8) une texture de surface lipophobe ou inversement.

6. Système de perfusion (1) selon l'une des revendications précédentes, **caractérisé en ce que** le cathéter (2) possède un adaptateur pour seringue (9).

7. Système de perfusion (1) selon l'une des revendications précédentes, **caractérisé en ce que** le cathéter (2) est conçu comme un produit à usage unique et le dispositif de commande (7) est conçu pour un usage multiple.
